# EUROPEAN PATENT APPLICATION

(11) **EP 1 939 164 A1**
(43) Date of publication of application: **02.07.2008**
(21) Application number: 06812040.1
(22) Date of filing: 19.10.2006
(51) Int. Cl.: C07C 37/20, C07C 39/16, C07B 61/00

(54) **PROCESS FOR PRODUCTION OF BISPHENOL A AND METHOD FOR JUDGMENT OF ION-EXCHANGE RESIN**

(30) Priority: 21.10.2005 JP 2005307649
(71) Applicant: IDEMITSU KOSAN CO., LTD., Tokyo 100-8321 (JP)
(72) Inventor: YOSHITOMI, Kazuyuki, Ichihara-shi, Chiba 2990193 (JP); KODAMA, Masahiro, Ichihara-shi, Chiba 2990193 (JP); MASUDA, Shuichi, Ichihara-shi, Chiba 2990193 (JP); KOHIRUIMAKI, Jun, Ichihara-shi, Chiba 2990193 (JP); YAMASAKI, Hokuto, Ichihara-shi, Chiba 2990193 (JP)
(74) Representative: Vossius & Partner
(86) International application number: PCT/JP2006/320862
(87) International publication number: WO 2007/046474

(57) **Abstract**

A process for the production of bisphenol A by condensing phenol with acetone in the presence of a strongly acidic ion-exchange resin, wherein an ion-exchange resin is judged to be a high-quality strongly acidic ion-exchange resin when three times as much deionized water at 25°C as ion-exchange resin is passed through the ion-exchange resin at an LHSV (liquid hour space velocity) of 1.0 hr⁻¹ to give an effluent exhibiting a pH of 5 or above at 25°C, and only such a high-quality strongly acidic ion-exchange resin is selected and used in the above production. According to the invention, a high-quality strongly acidic ion-exchange resin free from the eluation of residual sulfonic acid can be easily selected in the production of bisphenol A, which leads to stable production of bisphenol A with outstanding color tone. Further, the water consumption in washing a catalyst prior to the production of bisphenol A can be reduced, which leads to a lower wastewater disposal cost and stable long-period use of a catalyst.

## Description

### Technical Field

The present invention relates to a process for producing bisphenol A (2,2-bis(4-hydroxyphenyl) propane) and a method for rating an ion-exchange resin. More specifically, the present invention relates to a process for producing bisphenol A by condensing phenol with acetone using a strongly acidic ion-exchange resin catalyst, wherein the strongly acidic ion-exchange resin catalyst is selected so as to produce stably bisphenol A with outstanding color tone and a method for rating a high-quality, strongly acidic ion-exchange resin catalyst.

### Background Art

Bisphenol A is known to be an important compound as a raw material for engineering plastics such as polycarbonate and polyallylate, epoxy resin, and the like, and the demand for bisphenol A shows a tendency to grow more and more in recent years.
Bisphenol A is produced by condensing an excessive amount of phenol with acetone in the presence of an acid catalyst and optionally an auxiliary catalyst such as a sulfur compound. As the acid catalyst used in the above reaction, an inorganic mineral acid such as sulfuric acid and hydrochloric acid has conventionally been used but, in recent years, a strongly acidic ion-exchange resin has attracted attention and has been industrially used.

The strongly acidic ion-exchange resin contains residual sulfonic acid and is produced and supplied while the resin is swollen with water, so that the resin is unstable and is easily degraded. When the resin is used as a catalyst, there sometimes occurs a problem of catalyst alteration or degradation on storage or transportation.
For this reason, the strongly acidic ion-exchange resin containing the residual sulfonic acid is packed in a reactor, and then the resin is washed with pure water prior to the condensation between phenol and acetone (for example, see Patent Document 1).

However, the above method cannot prevent degradation of bisphenol A in quality and others, which is brought about by fluctuation of the quality of strongly acidic ion-exchange resins on the side of catalyst manufacturers. In other words, if the residual sulfonic acid is eluted from a strongly acidic ion-exchange resin during the production of bisphenol A, an adverse effect would be brought about on the quality of bisphenol A, but this elution of residual sulfonic acid is soon stopped in the case of a high-quality, strongly acidic ion-exchange resin. In the case of a low-quality strongly acidic ion-exchange resin, the elution continues longer. When bisphenol A is produced using the low-quality strongly acidic ion-exchange resin, it takes a long time before bisphenol A with outstanding color tone is produced. At the worst, a situation may arise where the strongly acidic ion-exchange resin as a catalyst has to be replaced by a new one before obtaining bisphenol A with outstanding color tone. The quality of the strongly acidic ion-exchange resin is determined in the production process thereof on the side of the catalyst manufacturers. However, the catalyst manufacturers have no appropriate method of rating the resin quality, so that the production plant of bisphenol A has to be operated under unstable conditions.

In the process described in Patent Document 1, the resin is washed with deionized water until the specific conductivity lowers below a specified value. However, when a low-quality strongly acidic ion-exchange resin shares a large portion of the resin, the resin is washed with a large amount of water, thereby producing a large amount of polluted waste water.
Further, when bisphenol A is produced in the mixed state of a good-quality strongly acidic ion-exchange resin and a low-quality strongly acidic ion-exchange, bisphenol A with outstanding color tone is not obtained and the catalyst life is shortened, thereby increasing the amount of wasted catalyst to be disposed.
Patent Document 1: Japanese Patent Application Laid-Open No. H09-173858

### Disclosure of the Invention

### Problems to be Solved by the Invention

In view of the above circumstances, on producing bisphenol A by condensing phenol with acetone using a strongly acidic ion-exchange resin catalyst, it is an object of the present invention to provide a process for stably producing bisphenol A with outstanding color tone by selecting in advance a strongly acidic ion-exchange resin that elutes no residual sulfonic acid.

### Means for Solving the Problems

The present inventors have made intensive studies in order to achieve the foregoing object, and found that the object can be fulfilled by rating the performance of a strongly acidic ion-exchange resin based on the pH of the water discharged after a specific amount of deionized water is passed through a sample of a strongly acidic ion-exchange resin catalyst produced by a catalyst manufacturer, and selecting only a high-quality, strongly acidic ion-exchange resin for use in the bisphenol A production plant. The present invention has been accomplished based on these findings.

Namely, the present invention provides a process for producing bisphenol A and a method for rating an ion-exchange resin in the following.
1. A process for producing bisphenol A by condensing phenol with acetone in the presence of a strongly acidic ion-exchange resin by using the strongly acidic ion-exchange resin that generates discharge water having the pH of 5 or more at 25°C, when deionized water at 25°C in an amount of 3 times the volume of the ion-exchange resin is passed through the resin at an LHSV (liquid hourly space velocity) of 1.0 hr⁻¹.

2. A process for producing bisphenol A by condensing phenol with acetone in the presence of a strongly acidic ion-exchange resin comprising the steps of:
   (1) selecting a high-quality, strongly acidic ion-exchange resin by measuring the free acid concentration of a strongly acidic ion-exchange resin based on the pH of water discharged when deionized water is passed through the strongly acidic ion-exchange resin;
   (2) washing the strongly acidic ion-exchange resin with deionized water and phenol after packing the high-quality, strongly acidic ion-exchange resin thus selected in a reactor for condensing phenol with acetone; and
   (3) condensing phenol with acetone using the strongly acidic ion-exchange resin thus washed.

3. The process for producing bisphenol A as described in 2 by selecting a strongly acidic ion-exchange resin that generates discharge water having the pH of 5 or more at 25°C, when deionized water at 25°C in an amount of 3 times the volume of the ion-exchange resin is passed through the resin at an LHSV (liquid hourly space velocity) of 1.0 hr⁻¹ in the foregoing step (1) of selecting a strongly acidic ion-exchange resin.
4. A method for rating an ion-exchange resin by rating an acidic ion-exchange resin that generates discharge water having the pH of 5 or more at 25°C, when deionized water at 25°C in an amount of 3 times the volume of the ion-exchange resin is passed through the resin at an LHSV (liquid hourly space velocity) of 1.0 hr⁻¹ as a strongly acidic ion-exchange resin.

### Effect of the Invention

According to the method for rating an ion-exchange resin of the present invention, it is quite easy to classify strongly acidic ion-exchange catalyst samples produced by a catalyst manufacturer and others into high-quality and low-quality samples. In the bisphenol A production plant, bisphenol A with outstanding color tone can be produced by selecting and using a high-quality resin for the condensation of phenol with acetone.
Further, according to the process for producing bisphenol A of the present invention, the amount of water used to wash the catalyst before the production of bisphenol A is reduced, so that the cost for waste water treatment can be minimized and the catalyst can be stably used for a long time.

### Best Mode for Carrying Out the Invention

The process for producing bisphenol A of the present invention is a process for producing bisphenol A by condensing phenol with acetone using a strongly acidic ion-exchange resin as a catalyst. In particular, a strongly acidic sulfonic acid-type ion-exchange resin is preferably used in view of the catalytic activity and others.
As the strongly acidic ion-exchange resin with sulfonic acid groups, there may be mentioned, for example, sulfonated styrene/divinylbenzene copolymer, sulfonated cross-linked styrene polymer, phenol-formaldehyde-sulfonic acid resin, benzene-formaldehyde-sulfonic acid resin, and the like.

In the present invention, there may be used preferably a strongly acidic ion-exchange resin that is partially modified with sulfur compounds or sulfur-containing amine compounds. The sulfur compounds that are used for the partial modification include alkylmercaptans such as methylmercaptan and ethylmercaptan. Examples of the sulfur-containing compounds include mercapto alkylpyridines such as 3-mercaptomethylpyridine, 3-(2-mercaptoethyl)pyridine, and 4-(2-mercaptoethyl)pyridine; mercapto alkylamines such as 2-mercaptoethylamine, 3-mercaptopropylamine, and 4-mercaptobutylamine; thiazolidines such as thiazolidine, 2,2-dimethylthiazolidine, 2-methyl-2-phenylthiazolidine, and 3-methylthiazolidine; aminothiophenols such as 4-aminothiophenol; and the like. These sulfur-containing amine compounds may be in a free form, or in a form of an adduct salt of an acidic substance such as hydrochloric acid, a quaternary ammonium salt or the like.

In the process for producing bisphenol A of the present invention, is used a high-quality, strongly acidic ion-exchange resin that is rated by the measurement of free acid concentration in the water discharged after a specific amount of deionized water is passed through a strongly acidic ion-exchange resin.
For this purpose, firstly strongly acidic ion-exchange resin samples produced generally by a catalyst manufacturer are rated, then only high-quality, strongly acidic ion-exchange resins are preferably selected and used as a catalyst for the production of bisphenol A. A sample of strongly acidic ion-exchange resins is rated based on the pH of the water discharged after a specific amount of deionized water is passed through the sample.

The criteria for rating the high-quality, strongly acidic ion-exchange resin are as follows: a resin is rated as a high-quality, strongly acidic ion-exchange resin, if the resin generates discharge water having the pH of 5 or more, preferably 5 to 7, and more preferably 6 to 7 at 25°C when deionized water at 25°C in an amount of 3 times the volume of the ion-exchange resin is passed through the resin at an LHSV (liquid hourly space velocity) of 1.0 hr⁻¹. The resin is generally rated as a low-quality ion-exchange resin when the pH of the discharge water is less than 5 after deionized water in an amount that is 3 times the resin is passed through the resin. Elution of sulfonic acid continues in the case of the low-quality strongly acidic ion-exchange resin, so that it takes a long time before bisphenol A with outstanding color tone is produced. At the worst, the strongly acidic ion-exchange resin as a catalyst has to be replaced by a new one before obtaining bisphenol A with outstanding color tone.

The above criteria for rating may be changed in accordance with the situation and the like of the strongly acidic ion-exchange resin samples supplied. For example, in the case of requiring urgent rating, rating of a resin as a good-quality, strongly acidic ion-exchange resin may be performed by reducing the amount of deionized water passing through the strongly acidic ion-exchange resin, where a pH of slightly lower than 5 may be obtained. However, in order to rate the quality of a strongly acidic ion-exchange resin accurately, the resin is rated by the pH of the discharge water obtained when deionized water at 25°C in an amount that is 3 times of the resin is passed through the resin.

When bisphenol A is produced by reacting phenol and acetone, a high-quality, strongly acidic ion-exchange resin selected as mentioned above is packed in a reactor; and the reactor is washed by passing through deionized water, and then by passing through phenol. When the strongly acidic ion-exchange resin is washed, the LHSV (liquid hourly space velocity) of water is 0.1 to 6 hr⁻¹, the LHSV of phenol is 0.05 to 5 hr⁻¹, the washing time with water is about 1 to about 48 hrs, and the washing time with phenol is about 1 to about 48 hrs.

In the present invention, after the high-quality, strongly acidic ion-exchange resin is selected and washed with deionized water and phenol, bisphenol A is produced by condensing phenol with acetone.
The ratio of phenol to acetone used for the production of bisphenol A is not particularly limited, but the amount of the unreacted acetone is desirably as small as possible in view of easiness of purification of bisphenol A produced, production cost, and others. Therefore, it is advantageous to use phenol in excess of the stoichiometric amount. Generally, phenol is used in an amount of 3 to 30 mol and preferably 5 to 20 mol per 1 mol of acetone. Further, in this production of bisphenol A, a reaction solvent is usually not required except when the viscosity of the reaction liquid is too high or when the reaction is carried out at such a low temperature that makes reactor operation difficult due to solidification.

For the condensation of phenol with acetone, can be employed a continuous fixed-bed reaction process where phenol and acetone are continuously fed to a reactor packed with a strongly acidic ion-exchange resin. One reactor may be used, or two or more reactors may be used in series or parallel. Industrially, it is particularly advantageous to connect two or more reactors packed with a strongly acidic ion-exchange resin in series and to employ a continuous fixed-bed multistage reaction process.

The reaction conditions for the continuous fixed-bed reaction process are explained. The molar ratio of acetone/phenol is selected in the range of generally 1/30 to 1/3 and preferably 1/20 to 1/5. When the molar ratio is smaller than 1/30, the reaction rate possibly becomes low: When the molar ratio is larger than 1/3, impurities are produced in a large amount and the selectivity for bisphenol A is likely to become lowered.
The reaction temperature is selected in the range of generally 40 to 150°C and preferably 55 to 100°C. At a reaction temperature lower than 40°C, not only the reaction rate is low but also the reaction liquid is possibly solidified in some cases because the viscosity of the reaction liquid is extremely high. At a reaction temperature exceeding 150°C, the control of the reaction is difficult, the selectivity for bisphenol A (p,p'-isomer) is lowered, and the strongly acidic ion-exchange resin serving as a catalyst is decomposed or degraded in some cases.
In the production of bisphenol A, the LHSV (liquid hourly space velocity) of the reaction mixture is generally 0.2 to 30 hr⁻¹ and preferably 0.5 to 20 hr⁻¹.

The reaction mixture in the reactor is subjected to a treatment in a conventional process, then bisphenol A is produced.
To give an example of the treatment, concentration is firstly carried out before crystallization. The concentration is usually carried out under the conditions including the temperature ranging from 130 to 170°C and the pressure ranging from 13 to 53 kPa. At a temperature lower than 130°C, a high vacuum is required. The temperature exceeding 170°C increases impurities or causes coloring. The bisphenol A concentration in the concentrated liquid is advantageously in the range of 25 to 40 % by mass. When the concentration is lower than 25 % by mass, the recovery percentage of bisphenol A is low. When the concentration exceeds 40 % by mass, slurry transportation becomes difficult after crystallization.

The concentrated liquid is generally cooled from 70-140°C to 35-60°C so as to crystallize a bisphenol A-phenol adduct, and the concentrated liquid changes into slurry. The crystallization of the bisphenol A-phenol adduct is carried out usually by vacuum cooling crystallization where the evaporation latent heat of water is used for cooling under vacuum. In the vacuum cooling crystallization, water is added in an amount of 3 to 20 % by mass to the concentrated liquid, and then crystallization is carried out under the conditions including the temperature ranging generally from 40 to 70°C and the pressure ranging from 3 to 13 kPa. When the amount of water added is less than 3 % by mass, heat removal is undesirably insufficient. When the amount exceeds 20 % by mass, the dissolution loss of bisphenol A becomes undesirably large. Further, when the crystallization temperature is lower than 40°C, the viscosity of the concentrated liquid possibly becomes large or the liquid is solidified. At a temperature exceeding 70°C, the dissolution loss becomes undesirably large.

The bisphenol A-phenol adduct crystallized in this way is separated in a conventional process, and then is usually washed with phenol. Subsequently, the washed adduct is decomposed into bisphenol A and phenol at the temperature of generally from 130 to 200°C and preferably from 150 to 180°C and under the pressure of generally from 3 to 20 kPa. The bisphenol A obtained through the above decomposition process is purified into bisphenol A with outstanding color tone by removing substantially all the phenol remaining therein through a process such as steam stripping. The bisphenol A is produced prill in a conventional process to obtain a product bisphenol A.

### Example

The present invention will be further described in detail with reference to the following examples, but it should be noted that the present invention is in no way limited by those examples.
Here, the color of the product bisphenol A was evaluated by visual inspection using the APHA standard color after the bisphenol A was heated in air at 220°C for 40 min.

### Example 1

Used as a catalyst is 200 cm³ of a sulfonic acid-type ion-exchange resin ("Diaion SK-104H" (trade name, manufactured by Mitsubishi Chemical Corporation) partly neutralized at 20 mol% with 2-mercapto ethylamine). The catalyst was packed in a washing pipe having an inside diameter of 26.4 mm and a height of 400 mm. Deionized water (25°C) in an amount of 600 cm³ (3 times of the catalyst) was passed through the washing pipe from the top thereof at an LHSV (liquid hourly space velocity) of 1.0 hr⁻¹, and the proton concentration (pH) originating from free acids was measured. The pH of the water discharged from the washing pipe was 6.5.
Next, the catalyst was packed in a fixed-bed reactor tower and washed with 1050 cm³ of deionized water (25°C). The pH after the water-washing was 6.5. Then, the catalyst was washed with 1450 cm³ of phenol, and the resulting waste water was analyzed. The sulfonic acid concentration after the washing with phenol was 0.1 ppm by mass.
Through the fixed-bed reactor tower packed with the catalyst, phenol and acetone in a molar ratio of 10:1 were continuously passed at an LHSV of 3 hr⁻¹. The reaction was performed at 75°C. The resulting reaction mixture liquid was concentrated in a vacuum distillation tower at 170°C and 66 kPa by distilling out acetone, water, and phenol.

Subsequently, water was added to the resulting phenol-bisphenol A solution. The resulting mixture was then cooled to 45°C in a crystallization drum to crystallize a bisphenol A-phenol adduct, and a slurry solution was obtained.
The resulting slurry solution was subjected to solid-liquid separation using a solid-liquid separating machine and washed with phenol, to obtain bisphenol A-phenol adduct crystals. Phenol was added to the adduct crystals, and the resulting mixture was heated to 170°C in a dissolution tank to prepare a solution containing phenol and bisphenol A. The solution was again subjected to cooling crystallization, solid-liquid separation, and phenol washing using an apparatus similar to the above, and purified crystals of bisphenol A and phenol adduct were obtained.
The purified adduct crystals were melted in a melting tank and were dephenolized in a distillation tower to obtain a bisphenol A melt. The melt was produced prill in a prilling tower in an inert gas atmosphere to obtain bisphenol A prill. The color of the resulting bisphenol A was evaluated to be APHA 5.

### Example 2

Bisphenol A was produced using a catalyst that generated discharge water having the pH of 5.5, which was obtained by the same rating method as used in Example 1.
The catalyst was packed in a fixed-bed reactor tower and washed with 1400 cm³ of deionized water (25°C). The pH after the water-washing was 6.1. Subsequently, the catalyst was washed with 1550 cm³ of phenol. The sulfonic acid concentration after the washing with phenol was 0.2 ppm by mass.
In the same way as in example 1, condensation and others of phenol with acetone were carried out. APHA of the resulting bisphenol A was 10.

### Comparative Example 1

Bisphenol A was produced using a catalyst that generated discharge water having the pH of 4.5, which was obtained by using the dame rating method as in Example 1.
The catalyst was paced in a fixed-bed reactor tower and washed with 1050 cm³ of deionized water (25°C). The pH after the water-washing was 4.2. Because the pH after the water-washing was low and sulfonic acid still eluted, water-washing was further continued. As a result, the pH became 5.2 when the amount of deionized water used was 2100 cm³, 5.5 at 3500 cm³, and still 5.5 at 7000 cm³. As above, in the case of this catalyst, the pH was not elevated no matter how many times water washing was repeated. Subsequently, the catalyst was washed with 1450 cm³ of phenol. After the washing with phenol, the sulfonic acid concentration was 2.7 ppm by mass.
In the same way as in example 1, condensation and others of phenol with acetone were carried out. APHA of the resulting bisphenol A was 30.
As described above, in the case of a poor-quality catalyst, the pH is not elevated no matter how many times water washing is repeated. If such a poor-quality catalyst is water-washed in a commercial plant, a large amount of polluted water is produced. Therefore, such a poor-quality catalyst has to be discarded. When the above results are converted to an actual industrial plant having a production capacity of several tens of thousands of tons, several tens of cubic meter catalyst would be discarded. In addition, no products are produced during this period, thereby causing an enormous loss.

### Comparative Example 2

Bisphenol A was produced using a catalyst that generated discharge water having the pH of 4.8, which was obtained by using the same rating method as in Example 1.
The catalyst was packed in a fixed-bed reactor tower and washed with 1050 cm³ of deionized water (25°C). The pH after the water washing was 4.8. Because the pH after the water washing was low and sulfonic acid was still eluted, water washing was further continued. As a result, the pH became 6.0 when the amount of deionized water used was 2100 cm³, 6.3 at 3500 cm³ and 6.5 at 7000 cm³. Subsequently, the catalyst was washed with 1450 cm³ of phenol. The sulfonic acid concentration after the washing with phenol was 0.1 ppm by mass.
In the same way as in example 1, condensation and others of phenol with acetone were carried out. APHA of the resulting bisphenol A provided was 15.
As described above, in order to improve a poor-quality catalyst into a catalyst that provides a product of good quality, a large amount of water is required for washing, whereby a large amount of polluted water is produced. Even though a large amount of water is used for washing, bisphenol A with outstanding color tone as in Example 1 cannot be produced.

### Industrial Applicability

According to the method for rating an ion-exchange resin of the present invention, a sample of a strongly acidic ion-exchange resin catalyst produced by a catalyst manufacture and others can be quite easily classified into high-quality and low-quality resins. In a bisphenol A production plant, bisphenol A with outstanding color tone can be stably produced by selecting and using a high-quality catalyst for the condensation of phenol with acetone.
Further, according to the process for producing bisphenol A of the present invention, the amount of water used to wash the catalyst can be minimized, so that the cost for wastewater treatment can be reduced and the catalyst can be used stably for a long time.

## Claims

1. A process for producing bisphenol A by condensing phenol with acetone in the presence of a strongly acidic ion-exchange resin comprising:
using the strongly acidic ion-exchange resin that generate discharge water having a pH of 5 or more at 25°C, when deionized water at 25°c in an amount of 3 times the volume of the ion-exchange resin is passed through the resin at an LHSV (liquid hourly space velocity) of 1.0 hr⁻¹.

2. A process for producing bisphenol A by condensing phenol with acetone in the present of a strongly acidic ion-exchange resin, comprising the steps of:
(1) selecting a high-quality, strongly acidic ion-exchange resin by measuring the free acid concentration of a strongly acidic ion-exchange resin based on the pH of water discharged when deionized water is passed through the strongly acidic ion-exchange resin;
(2) washing the strongly acidic ion-exchange resin with deionized water and phenol after packing the high-quality, strongly acidic ion-exchange resin thus selected in a reactor for condensing phenol with acetone; and
(3) condensing phenol with acetone using the strongly acidic ion-exchange resin thus washed.

3. The process for producing bisphenol A according to claim 2, wherein selected is a strongly acidic ion-exchange resin that generates discharge water having the pH of 5 or more at 25°C when deionized water at 25°C in an amount of 3 times the volume of the ion-exchange resin is passed through the resin at an LHSV (liquid hourly space velocity) of 1.0 hr⁻¹ in the foregoing step (1) of selecting a strongly acidic ion-exchange resin.

4. A method for rating an ion-exchange resin comprising rating a strongly acidic ion-exchange resin that generates discharge water having the pH of 5 or more at 25°C, when deionized water in an amount of 3 times the volume of the ion-exchange resin is passed through the resin at an LHSV (liquid hourly space velocity) of 1.0 hr⁻¹ as a high-quality, strongly acidic ion-exchange resin.
